# EUROPÄISCHE PATENTANMELDUNG

(11) **EP 1 248 107 A2**
(43) Veröffentlichungstag der Anmeldung: **09.10.2002**
(21) Anmeldenummer: 01118669.9
(22) Anmeldetag: 03.08.2001
(51) Int. Cl.: G01N 33/50, C12M 1/34

(54) **Verfahren und Vorrichtung zum zeitaufgelösten Screening**

(30) Priorität: 22.03.2001 DE 10114260
(71) Anmelder: IPF Pharmaceuticals GmbH, 30625 Hannover (DE)
(72) Erfinder: Motzkus, Dirk, Dr. rer.nat., 30625 Hannover (DE); Leitlof, Rüdiger, 30625 Hannover (DE); Maronde, Erik, Dr. rer.nat., 30625 Hannover (DE)
(74) Vertreter: von Kirschbaum, Alexander, Dipl.-Ing.

(57) **Zusammenfassung**

Die Erfindung betrifft eine Vorrichtung, deren Verwendung und ein Verfahren zum zeitaufgelösten Screening, insbesondere von zellulären und biochemischen Prozessen.

## Beschreibung

Die Erfindung betrifft eine Vorrichtung, deren Verwendung und ein Verfahren zum zeitaufgelösten Screening, insbesondere von zellulären und biochemischen Prozessen.

Bei der systematischen Suche nach Wirkstoffen haben neben der vergleichenden Sequenzanalyse in den letzten Jahren in der biochemischen und zellbiologischen Forschung Screeningverfahren sehr an Bedeutung gewonnen. Dazu zählen die Array- und Chiptechnologie. Insbesondere in der pharmazeutischen Forschung sind Verfahren etabliert worden, die es erlauben, in relativ kurzer Zeit eine Vielzahl verschiedener potentieller Wirkstoffe einem Ziel (Target) zuzuführen (High Throughput Screening, HTS). Eine spezifische Interaktion wird durch ein Signal, wie zum Beispiel die Emission von Photonen, angezeigt. Bei einem effektiven Screeningverfahren können so ganze Substanzbibliotheken "durchgemustert" werden, wobei das Verfahren meist automatisiert durchgeführt wird.

Ein Gebiet der Biochemie, das zur Zeit intensiv beforscht wird, ist der zirkadiane Rhythmus des Organismus. Die Anpassung an temporale Veränderungen, wie z.B. dem Wechsel von Tag und Nacht, stellt eine fundamentale Eigenschaft aller höheren Lebewesen dar. Mit Hilfe biologischer Uhren ist es möglich, auch bei vollständiger Dunkelheit das Auf- und Untergehen der Sonne abzuschätzen. Die Periodenlänge dieses autonom generierten Rhythmus beträgt ca. 24 Stunden und wird als "zirkadian" bezeichnet. Für die Regulation des zirkadianen Rhythmus sind drei wesentliche Komponenten notwendig: Erstens können externe Stimuli die Periodenlänge des Oszillators verändern (afferenter "Input-pathway"). Zweitens muss der interne Rhythmus im zentralen Zeitgeber persistieren. Drittens muss die zirkadiane Information an die Peripherie weitergegeben werden (efferenter "Output-pathway").

An der Generierung und Rückkopplung des zirkadianen Rhythmus sind unter anderem die Proteine CLOCK, BMAL1, Period und Cryptochrom beteiligt. Heterodimere von CLOCK und BMAL1 aktivieren die Transkription der Gene für Period (Per), Cryptochrom (Cry) und weiterer Uhr-kontrollierter Gene ("clock-controlled genes", CCGs). Durch die Interaktion der entstehenden Proteine Period und Cryptochrom wird eine negative Rückkopplung erzeugt. Die Anpassung des Rhythmus an externe Stimuli (z.B. Licht) erfolgt über afferente Signalwege. Die Information über die Phase innerhalb des SCN wird über efferente Bahnen an die "Slave"-Oszillatoren weitergegeben.

Der zentrale Vermittler des zirkadianen Rhythmus befindet sich in einem paarigen Kern des ventralen Hypothalamus, dem Nucleus Suprachiasmaticus (SCN). Durch Zerstörungs- und Transplantationsexperimente konnte gezeigt werden, daß der SCN die übergeordnete Struktur ("Master"-Oszillator) für die Vermittlung des Rhythmus darstellt. Beim molekularen Prozess der Rhythmus-Generierung bilden die Genprodukte von Period (Per) und Cryptochrom (Cry) (Inhibitoren) eine negative Rückkopplung zu den Aktivatoren Clock und Bma1 aus. Die Phase des autonom generierten Rhythmus wird vom SCN ausgehend über neuronale (van Essefeld et al., 2000) und humorale Wege (Silver et al., 1996) an die Peripherie weitergegeben. Neuere Arbeiten deuten daraufhin, dass die zentralen Mechanismen der zirkadianen Oszillation für Gewebe (Yamazaki et al., 2000) wie auch für Zellen in Kultur (Balsalobre et al., 1998; Sakamoto et al., 1998; Oishi et al., 1998) gelten. In dem hierarchischen System scheint der SCN als "Master" die Aufgabe eines Zeitgebers zu übernehmen, der über bisher unbekannte Wege alle "Slave"-Oszillatoren der peripheren Gewebe synchronisiert. Hierbei scheint Period 1 (Per1) eine Schlüsselrolle einzunehmen. Innerhalb des SCN konnte gezeigt werden, dass ein Lichtpuls innerhalb der subjektiven Nacht zu einer sehr schnellen und starken Induktion des Gens für Per1 führt (Albrecht et al., 1997 und 2001). Intracerebroventrikuläre Injektion von Per1-Antisense Oligonukleotiden führt zur Reduktion einer durch Licht induzierten Phasenverschiebung (Akiyama et al., 1999), so dass Per1 wahrscheinlich am Vorgang der Zurückstellung (resetting) der Uhr beteiligt ist. In funktionellen mPer1 Mutanten konnte ebenfalls gezeigt werden, daß Per1 für die Anpassung des Rhythmus verantwortlich ist. Weiterhin konnte in einem humanen Zellmodell die Induktion von hPER1 durch die Aktivierung von PKA- und PKC abhängigen Signaltransduktionswegen initiiert werden (Motzkus et al., 2000). Somit scheint die Induktion von hPER1 für die Verschiebung der Phase sowohl im "Master" als auch in "Slave"-Oszillatoren eine Schlüsselrolle einzunehmen.

Während die Mechanismen des zentralen "Schrittmachers" innerhalb des SCN in den letzten Monaten sehr ausführlich untersucht wurden, ist die Regulation der "Slave"-Oszillatoren in der Peripherie weitgehend unbekannt. Yamazaki et al. (2000) zeigten die Abhängigkeit der peripheren Rhythmen vom SCN durch die Generierung transgener Ratten. Durch die Fusion des Per1-Promotors an ein Luziferase-Reportergen konnte in kultiviertem SCN über 62 Tage ein persistierender zirkadianer Rhythmus der Genexpression detektiert werden. Darüber hinaus konnte in explantiertem Gewebe von Skelettmuskel, Leber und Lunge unter statischen Bedingungen ebenfalls Rhythmizität gemessen werden. Dabei betrug die Verschiebung der "Slave" Oszillatoren der nach in vivo-Befunden erwarteten Zeitverschiebung von 3-9 Stunden. Im Gegensatz zur persistierenden Rhythmik von SCN-Zellen zeigten die anderen explantierten Gewebskulturen eine stete Abnahme der Amplitude des Reporters. Die durch Mediumwechsel initiierte Resynchronisation der Zellen konnte die Abschwächung der Rhythmik kompensieren bzw. neue Rhythmizität auslösen. Weiterhin zeigte die Arbeit von Yamazaki et al., daß nach einer durch Licht induzierten Phasenverschiebung SCN-Zellen ihren Rhythmus spontan regulieren, während periphere Organkulturen eine stark verzögerte Anpassung zeigten. Dieser Effekt spiegelt dem Anschein nach genau den Sachverhalt *in vivo* wieder, nämlich dass nach Durchreisen von Zeitzonen und dem eintretenden "Jet-lag" die Physiologie des Körpers erst nach einiger Zeit (ca. 1 Woche) synchronisiert wird.

Diese und andere Arbeiten unterstützen die Hypothese, daß der Organismus durch ein hierarchisch angelegtes zirkadianes System gesteuert wird. Der SCN ist hierbei die oberste Instanz, die den entscheidenden Impuls zur Aufrechterhaltung und Anpassung der peripheren "slave" Oszillatoren gibt.

Trotz intensiver Bemühungen zur Aufklärung der intrinsischen Mechanismen der zentralen Uhr, deren Komponenten auch die peripheren Oszillatoren steuern, sind keine "Rhythmus-übertragenden" Faktoren bekannt, die die Informationen vom SCN zur Peripherie weiterleiten.

Ein Verfahren zur Ermittlung von Faktoren, die den zirkadianen Rhythmus von Zellen beeinflussen, wird in der noch nicht veröffentlichten Patentanmeldung PCT/EP/0104383 beschrieben. Dabei wird ein transfizierbares Konstrukt aus dem Promotor des humanen Period1-Gens und Luziferase verwendet. Dieses Konstrukt wird als hPER1-luc bezeichnet. Das Konstrukt wird in eine menschliche Zelllinie transient oder stabil transfiziert. Diese Zellen können dann in Gegenwart von Luziferin mit Testsubstanzen in Kontakt gebracht werden. Die dann messbare Chemilumineszenz ist proportional zur Expression der Luziferase und zeigt damit die Wirkung der getesteten Substanzen auf den Promotor des hPER1. Das hPER1-luc Konstrukt kann in menschlichen Zelllinien (z.B. HUH-7, SNB-19, U2-05 oder SH-SY5Y) transient oder stabil transfiziert werden. Die transfizierten Zellen werden dabei auf 96 Well Platten mit weißen Wänden ausgesäht und über Nacht stehen gelassen. Am nächsten Tag werden die Zellen mit Medium, welches 10-1000 µM Natrium-Luziferin enthält, mit den entsprechenden Kontrollsubstanzen oder Peptidfraktionen versetzt. Die Platte wird alle 1 bis 4 Stunden in einem Chemilumineszenzlesegerät über bis zu drei Tage vermessen. Zwischen den Messungen wird die Platte in einen Inkubator gestellt.

Das Verfahren nach PCT/EP/0104383 ist eine wichtige Methode zum Nachweis von Substanzen, die den zirkadianen Rhythmus beeinflussen. Wenn das Verfahren als Screeningverfahren verwendet wird, weist es jedoch einige Nachteile auf: So werden, wie beim HTS allgemein üblich, Zellen mit einer bestimmten Menge eines potentiellen Wirkstoffs versetzt und für eine bestimmte Zeit inkubiert. Während dieser Zeit verändert sich das Medium (zum Beispiel weil sich der Wirkstoff mit der Zeit aufbraucht; pH-Änderungen) so dass sich die Zellen nach einiger Zeit in einer veränderten Umgebung befinden. Bei Prozessen wie dem zirkadianen Zyklus werden zeitlich bedingte Veränderungen durchlaufen, weswegen Änderungen der Medienbedingungen einen profunden Einfluß auf den zirkadianen Rhythmus haben können (Yamazaki et al., 2000). Daher ist das Verfahren der PCT/EP/0104383 als Screeningverfahren nur mit beschränktem Erfolg verwendbar.

Allgemein liegt bei der Suche nach Faktoren oder Wirkstoffen, die den zirkadianen Rhythmus steuern, das grundlegende Problem vor, das übliche Verfahren des High-Throughput Screening nicht oder nur bedingt geeignet sind. Dabei wird nämlich zu einem Target ein potentieller Wirkstoff gegeben, und das Auftreten eines Signals zu einzelnen Zeitpunkten beobachtet. Ein gesuchter "Rhythmusübertragender" Faktor würde den Rhythmus jedoch nicht aus- oder einschalten, sondern einen Impuls zur Änderung des Rhythmus geben. Bei Prozessen wie dem zirkadianen Rhythmus ist die Konzentration des Rhythmus-beeinflussenden Faktors von großer Bedeutung. Viele Komponenten des Systems der inneren Uhr sind zyklisch wirksam, was von Konzentrationsänderungen dieser Komponenten, der wirksamen Form dieser Komponenten (z.B. geändert durch Phosphorylierung) oder der Interaktion mit anderen Komponenten der Uhr herrührt.

Der Erfindung liegt somit die Aufgabe zugrunde, eine Vorrichtung und ein Verfahren zu entwickeln, mit denen ein effektiveres Screening von Faktoren und Wirkstoffen möglich ist, die die oben genannten Prozesse beeinflussen und steuern. Die Vorrichtung sollte es ermöglichen, für eine Vielzahl von Proben oder Wirkstoffen gleichzeitig zu testen, ob diese in den beobachteten Systemen zeitliche Anpassungen oder Veränderungen hervorrufen. Insbesondere sollte ein Verfahren bereitgestellt werden, dass ein effektives Screening von potentiellen Faktoren und Wirkstoffen ermöglicht, die den zirkadianen Rhythmus steuern oder beeinflussen.

Die Lösung der Aufgabe erfolgt erfindungsgemäß durch eine Vorrichtung gemäß Anspruch 1, deren Verwendung gemäß Anspruch 9 und durch ein Verfahren gemäß Anspruch 12.

Die erfindungsgemäße Vorrichtung, die insbesondere zur Durchführung des vorstehend beschriebenen Verfahrens geeignet ist, weist einen in einer Dunkelkammer angeordneten Probenträger mit einer Vielzahl von Vertiefungen auf. Bei dem Probenträger handelt es sich beispielsweise um eine Titerplatte mit 96, 386 oder mehr Vertiefungen (Wells). Ferner weist die erfindungsgemäße Vorrichtung Verschlußeinrichtungen zum Verschließen der Vertiefungen auf, wobei jede Verschlußeinrichtung, durch die ein Well verschlossen ist, eine mit der entsprechenden Vertiefung verbundene Einlass- und eine Auslassöffnung aufweist. Bei den Verschlußeinrichtungen kann es sich um einzelne stempelartige, im wesentlichen zylindrische Verschlußeinrichtungen handeln, die jeweils ein Well gesteckt werden. Auch können mehrere Verschlußeinrichtungen miteinander verbunden sein, so dass mehrere Wells gleichzeitig verschlossen werden können. Es ist ebenso möglich, sämtliche Verschlußeinrichtungen, die z.B. sämtliche Vertiefungen eines Probenträgers vorgesehen sind, miteinander zu verbinden, so dass ein Deckel ausgebildet ist, durch den sämtliche Vertiefungen gleichzeitig verschlossen werden. Wesentlich ist hierbei, dass die Verschlußeinrichtungen bzw. der Deckel derart ausgebildet ist, dass für jede Vertiefung eine Einlaßöffnung und eine Auslaßöffnungen vorgesehen ist. Hierdurch ist es möglich, dem Well ein Medium zu- und abzuführen, so dass beispielsweise ein kontinuierlicher Mediumaustausch verwirklicht werden kann.

Ferner weist die erfindungsgemäße Vorrichtung eine in der Dunkelkammer angeordnete Meßeinrichtung, beispielsweise eine CCD-Kamera, zum Messen der von den Zellen abgegebenen Photonen auf.

Die erfindungsgemäße Vorrichtung weist insbesondere den Vorteil auf, dass in jedem einzelnen Well Untersuchungen, insbesondere die anhand des vorstehend beschriebenen Verfahrens beschriebenen Untersuchungen, durchgeführt werden können. Es ist somit beispielsweise bei einer 96er Titerplatte möglich, gleichzeitig 96 unterschiedliche Reaktionen der Zellen zu untersuchen. Insbesondere kann die Untersuchung bei physiologischen Bedingungen und unter kontinuierlichem Fluss eines Mediums durch die Wells erfolgen, um zeitaufgelöst Genaktivitäten in den Zellen zu messen, die sich auf Grund der Zugabe von Substanzen verändern können.

Vorzugsweise ist in der Dunkelkammer eine Halteeinrichtung, wie beispielsweise ein Meßtisch, vorgesehen. In einer Halteeinrichtung kann der Probenträger derart angeordnet werden, dass er bezüglich der Meßeinrichtung lagegenau gehalten ist. Es ist somit eine exakte Zuordnung einzelner Wells möglich. Insbesondere ist es möglich, jeweils ein Well einer vorgegebenen Anzahl von Pixeln der CCD-Kamera zuzuordnen. Die einzelnen Pixel können sodann von einer mit der CCD-Kamera verbundenen Auswerteeinrichtung ausgelesen werden. Von der Auswerteeinrichtung erfolgt beispielsweise ein Integrieren der wahrgenommenen Photonen eines Wells in festen Zeitabständen.

Vorzugsweise ist die Meßeinrichtung auf der den Verschlußeinrichtungen abgewandten Seite des Probenträgers angeordnet. Üblicherweise ist die Meßeinrichtung somit unterhalb des Probenträgers angeordnet, so dass die von den Proben abgegebenen Photonen durch den Boden des Probenträgers hindurch zur Meßeinrichtung abgegeben werden.

Bei einer besonders bevorzugten Ausführungsform ist jede Einlaßöffnung und jede Auslaßöffnung einer Verschlußeinrichtung über ein gesondertes Fluidelement, wie einen Schlauch, mit einer Medium-Fördereinrichtung verbunden. Es ist somit möglich, einen Teil der mit den Einlass- bzw. Auslaßöffnungen verbundenen Fluidelemente miteinander zu verbinden und durch die entsprechenden Wells dasselbe Medium hindurchzuleiten. Ebenfalls ist es möglich, sämtliche Einlass-Fluidelemente und/oder Auslass-Fluidelemente miteinander zu verbinden. Es ist jedoch wahlweise auch möglich, jedes Well mit einem Medium zu versorgen.

Nachfolgend wird die Erfindung anhand einer bevorzugten Ausführungsform unter Bezugnahme auf die anliegenden Zeichnungen näher erläutert.

Es zeigen:

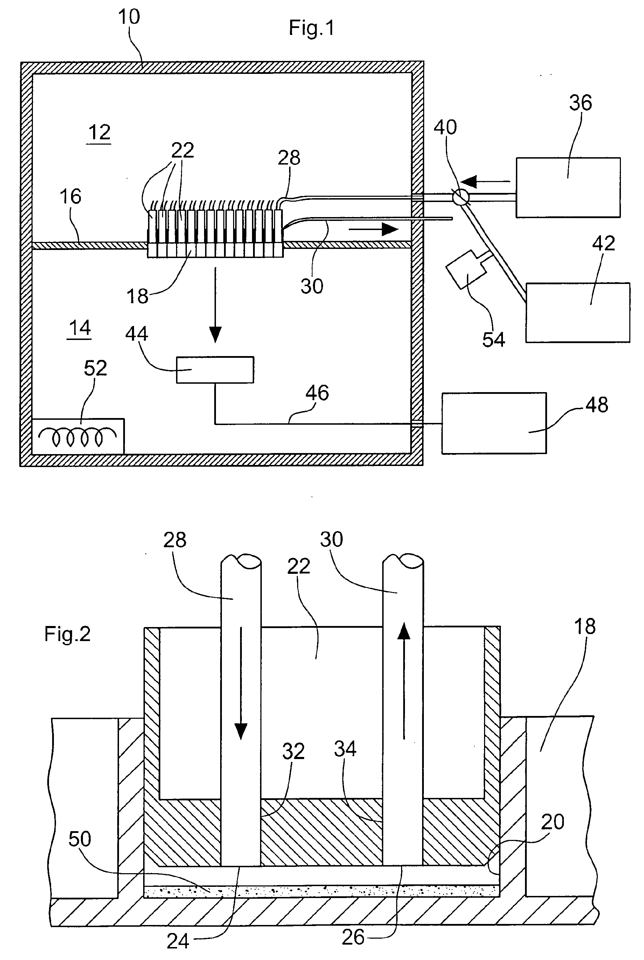

- Fig. 1: eine schematische Schnittansicht einer bevorzugten Ausführungsform der erfindungsgemäßen Vorrichtung und
- Fig. 2: eine vergrößerte Schnittansicht eines mit einer erfindungsgemäßen Verschlußeinrichtung verschlossenen Wells einer Titerplatte.

In einer Dunkelkammer 10 ist eine die Dunkelkammer 10 in eine obere und eine untere Kammer 12 und 14 unterteilende Haltevorrichtung 16 vorgesehen. Die Haltevorrichtung 16 ist zum Halten einer oder mehrerer Probenträger 18, wie beispielsweise einer Titerplatte mit beispielsweise 96, 384 oder mehr Vertiefungen 20 (Wells), vorgesehen.

In jede der Wells 20 ist eine Verschlußeinrichtung 22 mit im wesentlichen kreiszylindrischem Querschnitt gesteckt. Hierzu ist der Außendurchmesser der Verschlußeinrichtung geringfügig kleiner als der Außendurchmesser der Wells 20. Um ein Abdichten zwischen der Verschlußeinrichtung 22 und des Wells 20 zu gewährleisten, kann ein Dichtring vorgesehen sein. Vorzugsweise sind die Abmessungen der Verschlußeinrichtungen so gewählt, dass ein Abdichten durch Reibung erfolgt und ein Dichtring entfallen kann. Hierzu kann das Well und/oder die Verschlußeinrichtung konisch ausgebildet sein.

Jede einzelne Verschlußeinrichtung 22 weist eine mit dem Well verbundene Einlaßöffnung 24 sowie eine Auslaßöffnung 26 auf. Die beiden Öffnungen 24,26 sind jeweils mit einem Fluidelement, wie einem Schlauch 28 bzw. 30, verbunden. Die Schläuche 28,30 sind durch Einstecken in Kanäle 32 bzw. 34 mit der Verschlußeinrichtung 22 verbunden. Gegebenenfalls kann ein Einkleben zum Fixieren der Schläuche 28,30 erfolgen.

Der Schlauch 28 wird aus der Dunkelkammer 10 herausgeführt und mit einer Pumpe 36, insbesondere eine peristaltischen Pumpe, verbunden. Die Verbindung mit der Pumpe, die mit einem oder mehreren nicht dargestellten Vorratsbehältern verbunden ist, kann gemeinsam erfolgen, so dass dasselbe Medium sämtlichen Wells 20 zugeführt wird. Ebenso können Gruppen von Schläuchen gebildet sein, die gemeinsam verbunden sind. Es ist ferner möglich, durch Vorsehen entsprechender Ventile oder anderer geeigneter Schaltvorrichtungen jeden Schlauch 28 gesondert mit der Pumpe 36 zu verbinden, so dass den einzelnen Wells 20 unterschiedliche Medien zugeführt werden können.

Über eine Ventileinrichtung 40 sind die Schläuche 28 einzeln oder gemeinsam mit einer weiteren Pumpe 42 verbunden. Durch Probenspritzen 100 können vorzugsweise einzeln oder gemeinsam durch die Schläuche 28 verschiedene Proben den einzelnen Vertiefungen 20 zugeführt werden. Die Pumpe 42 sorgt dabei sowohl für die Zufuhr der Substanzen aus den Probenspritzen 100 als auch für die Aufrechterhaltung des konstanten Drucks innerhalb des Systems.

Die von der Verschlußvorrichtung 22 abführenden Schläuche 30 können wahlweise in einen gemeinsamen Abführschlauch gebündelt oder einzeln, z.B. zur nachfolgenden Bestimmung von Zell-Metaboliten, getrennt aus der Dunkelkammer 10 ausgeführt werden.

Auf der den Verschlußeinrichtungen 22 gegenüberliegenden Seite des Probenträgers 18 ist eine Meßeinrichtung 44, beispielsweise eine CCD-Kamera, angeordnet. Im dargestellten Ausführungsbeispiel ist die CCD-Kamera 44 somit unterhalb der Halteeinrichtung 16 angeordnet. Mit Hilfe der CCD-Kamera 44 werden die einzelnen Wells 20 des Probenträgers 18 beobachtet. Die von den einzelnen Pixeln wahrgenommenen Photonen werden in elektrische Signale umgewandelt und über eine elektrische Leitung 46 an eine Auswerteeinrichtung 48 übermittelt. Die Pixel der CCD-Kamera 44 sind bezüglich der einzelnen Wells 20 vorzugsweise derart angeordnet, dass jeweils ein einzelnes Pixel oder eine Gruppe von Pixeln einem Well 20 zugeordnet ist. Hierdurch kann jedes einzelne Well 20 gesondert von der CCD-Kamera beobachtet und die abgegebenen Photonen der in einer Well angeordneten Zellen 50 gesondert wahrgenommen und ausgewertet werden.

Ferner ist in der Dunkelkammer 10 eine Heizeinrichtung 52 vorgesehen, die mit einem vorzugsweise steuerbaren Thermostat verbunden ist, um die Temperatur innerhalb der Dunkelkammer 10 über den gesamten Untersuchungszeitraum konstant zu halten. Ebenso ist es möglich, einen Temperaturgradienten aufzubringen.

Die Vorrichtung wird erfindungsgemäß zur zeitaufgelösten Untersuchung von Prozessen, insbesondere zellulären und biochemischen Pozessen, verwendet. Zu diesen zählen unter anderem die zelluläre Antwort auf äußere Stimuli, die zelluläre Antwort auf äußere Bedingungen, der zirkadiane Rhythmus von Zellen, Apoptose, die Expression von Genen, die Funktion von Promotorn, Änderungen von Protein/Protein-Wechselwirkungen, Phosphorylierung und Dephopsphorylierung von Signaltransduktionsmediatoren, Differenzierungs- oder Wachstumsprozesse, die Veränderungen von speziellen Markergenen für Differenzierungs- und Wachstumsprozesse, Proteindislokation, Änderungen von Proteinkonzentrationen in bestimmten Zellkompartimenten und/oder Proteintranslokation.

Gegenstand der Erfindung ist auch ein Verfahren zur zeitaufgelösten Untersuchung von zellulären und biochemischen Prozessen unter Verwendung einer Vorrichtung nach einem der Ansprüche 1 bis 8. Dabei werden in den Vertiefungen (20) Zellen positioniert, flüssige Medien durch die Einlaßöffnungen (24) den Vertiefungen (20) zugeführt und durch die Auslaßöffnungen (26) aus den Vertiefungen abgeführt, und es wird die Abgabe von Strahlung, insbesondere von Photonen, aus den Vertiefungen (20) mit Hilfe der Messeinrichtung (44) verfolgt. Vorzugsweise werden die Medien unter konstantem Medienfluß zugeführt. Das Verfahren der Erfindung wird gleichzeitig mit einer Vielzahl von Proben und vorzugsweise automatisiert durchgeführt. Das erfindungsgemäße Verfahren ist insbesondere geeignet zur Untersuchung der zellulären Antwort auf äußere Stimuli, der zellulären Antwort auf äußere Bedingungen, des zirkadianen Rhythmus von Zellen, der Apoptose, der Expression von Genen, der Funktion von Promotorn, Änderungen von Protein/Protein-Wechselwirkungen oder der Proteintranslokation.

Das Verfahren der Erfindung ist besonders geeignet, um zeitaufgelöst zu untersuchen, ob und wie in den Medien enthaltene Substanzen oder Substanzgemische zelluläre Prozesse auslösen oder beeinflussen. In einer bevorzugten Ausführungsform sind oder enthalten diese Substanzen oder Substanzgemische Peptide oder Peptidgemische. Geeignet sind insbesondere menschliche Peptidbanken, d.h. Substanzbibliotheken, die eine Vielzahl von Peptiden in teilweise oder vollständig aufgereinigten Fraktionen enthalten. Entsprechende Peptidbanken können Hemofiltratbanken oder Plazentabanken sein. Die Peptide oder Peptidgemische können vorzugsweise auch aus tierischen, insbesondere bovinen oder porcinen Geweben oder aus humanen oder tierischen Zelllinien oder deren Zellkulturüberständen isoliert worden sein. Die sukzessive Aufreinigung solcher Aktivitäten erfolgt nach etablierten Standardverfahren (Seiler et al., 1999), wodurch die entsprechenden Aktivitäten isoliert und identifiziert werden können. Dabei werden idealerweise moderne Analysemethoden wie MALDI-, CC-ESI-Massenspektroskopie oder CZE (Capillary Zone Electrophoresis) verwendet.

In einer bevorzugten Ausführungsform der Erfindung sind die Zellen mit einem Konstrukt aus dem Promotor des humanen Period1-Gens und einem Reportergen transfiziert und werden mit Substanzen oder Substanzgemischen inkubiert, wobei jeweils die Expression des Reportergens bestimmt wird. Unter diesen Bedingungen lassen sich Substanzen identifizieren, die den zirkadianen Rhythmus von Zellen beeinflussen. Besonders geeignet sind für solche Zwecke Zellen, die mit einem Konstrukt (hPER1-luc) aus dem Promoter des humanen Period1-Gens und dem Reportergen Luziferase transfiziert sind. Solche Systeme werden in der noch nicht veröffentlichten Patentanmeldung PCT/EP0104383 beschrieben, auf die hier ausdrücklich Bezug genommen wird.

Erfindungsgemäß können neben der schon erwähnten Luziferase, wie z.B. "Firefly" oder "Renilla" Luziferase, weitere geeignete Reportergene, gegebenenfalls in Kombination mit geeigneten Substraten, verwendet werden, wie intrazelluläre oder exkretierte alkalische Phosphatase, Grünes Fluoreszenz Protein oder Derivate des Proteins (Blaues, Gelbes, Rotes Fluoreszenz Protein), beta-Galactosidase (beta-Gal; lacZ) oder Chloramphenicol-Acetyltransferase (CAT). Diese werden mit dem PER1-Promoter oder je nach Verwendung mit einem anderem geeigeneten Promoter kombiniert.

Mit dem erfindungsgemäßen Verfahren können Substanzen identifiziert werden, die den zirkadianen Rhythmus des Menschen verzögern (Phasenverzögerer) oder beschleunigen (Phasenbeschleuniger).

Das erfindungsgemäße Verfahren ist insbesondere geeignet für die Identifizierung von schlafmodulierenden und neuroaktiven Substanzen in menschlichen Zelllinien, zum Screening von Substanzbibliotheken, insbesondere Hemofiltratbanken oder Plazentabanken, zum Screening von potentiellen Wirkstoffen gegen Schlaf-assozierte Erkrankungen, ausgewählt aus der Gruppe umfassend "Jet-Lag", Folgen von anhaltender Schichtarbeit und 'seasonal affective disorders' (SAD), Tag-Müdigkeit mit Schlafanfällen (hypersomnia), Schlafstörungen, Fettsucht, Kohlenhydrat-Begierde, Symptomen die der Überwinterung ähneln, Depressionen, Psychosen, Veränderungen der Melatonin- und Cortison-Sekretion, Verkürzung der REM-Schlaf-Phase und Erkrankungen der Physis und des Gemüts, die im Zusammenhang mit zirkadianen Störungen beim Menschen stehen.

Das erfindungsgemäße Verfahren unter Verwendung der erfindungsgemäßen Vorrichtung hat zahlreiche Vorteile gegenüber bekannten Verfahren:

Bei Verwendung der erfindungsgemäßen Vorrichtung können mit geringem Aufwand gleichzeitig 96 voneinander unabhängige Experimente oder mehr durchgeführt werden. Dabei können die Zellen permanent über einen längeren Zeitraum gleichbleibenden Bedingungen ausgesetzt werden. Bei diesen Experimenten lassen sich daher auf einfache Weise Bedingungen einstellen, bei denen Zellen unter weitgehend physiologischen Bedingungen kultiviert werden. Natürlich ist es auf der anderen Seite auch möglich, die Wirkung einer (langsamen) Konzentrationsänderung von Wirkstoffen zu beobachten, indem der Anteil des Wirkstoffes im Medium kontrolliert sinkt oder ansteigt.

Durch die erfindungsgemäße Simulation von physiologischen Bedingungen ist der Ersatz von Verfahrensschritten möglich, die bislang nur im Tierversuch durchgeführt werden konnten.

Ein weiterer Vorteil gegenüber bekannten Techniken und Tierversuchen ist es, dass die verwendeten Zellen humanen Ursprungs sind. Dadurch ist die Wahrscheinlichkeit, dass gefundene Wirkstoffe auch auf den ganzen Organismus wirken und als Arzneimittel verabreicht werden können, deutlich höher.

Besondere Bedeutung kommt der erfindungsgemäßen Verwendung eines humanen Systems in der molekularen Chronobiologie zu, weil bekanntermaßen zwar in verschiedenen Organismen homologe Proteine an der Generierung und Aufrechterhaltung des Rhythmus beteiligt sind, diese jedoch selbst in nahe verwandten Spezies unterschiedliche Funktionen übernehmen (van Essefeldt, 2000). Daher ist nur durch das verwendete humane System eine direkte Identifikation von Faktoren möglich, die zur Entwicklung von Pharmaka führen können.

Fast alle bisherigen Untersuchungen zirkadianer Mechanismen richten sich auf die fundamentalen Vorgänge innerhalb des SCN, seiner Regulation und den afferenten Signalwegen. Das hier verwendete Verfahren ermöglicht die Isolierung zirkulierender Faktoren, die in peripheren Organen Veränderungen der Periodenlänge (τ) verursachen. Ein solcher synchronisierender Faktor ist mit den bekannten Screening-Methoden kaum faßbar.

Die oben aufgeführten Vorteile wurden teilweise anhand des hPER1-luc Systems zur Ermittlung von Substanzen, die den zirkadianen Rhythmus steuern, erläutert, lassen sich aber für eine Vielzahl von Prozessen ausnutzen. Das erfindungsgemäße Verfahren erlaubt es, permanent (d.h., nicht nur an einzelnen Zeitpunkten), unter konstanten Bedingungen, gegebenenfalls automatisiert und über einen Zeitraum von Tagen eine Vielzahl von Systemen zu beobachten.

Ein weiterer Vorteil ist, dass erfindungsgemäß nicht nur geprüft werden kann, ob eine Substanz wirkt, sondern auch wann sie ihre Wirkung entfaltet.

### Ausführungsbeispiel:

Die im folgenden unter 1. bis 4. beschriebene Herstellung und Funktion eines hPER1-luc-Konstrukts und damit transfizierter Zellen wird auch in der PCT/EP/0104383 ausführlich beschrieben.

### 1. Konstruktion des hPER1-luc

Die Klonierung des hPER1-luc erfolgt ausgehend von dem Plasmid hPER1-SuperCos I (39C2; Sun et al., 1997). Das Cosmid wurde mit verschiedenen Restriktionsenzymen geschnitten und die resultierenden Banden mit einer PER1 Sonde hybridisiert. Das so identifizierte hPER1-HindIII Fragment wurde in pBluescript (pBRS) kloniert. Anschließend erfolgte eine Subklonierung in pEGFP-N1 (pEGFP), aus dessen Vektorsequenz die NheI-Schnittstelle genutzt wurde. Dieser Vektor, ebenso wie der promoterlose pGL2-basic Vektor, wurden so modifiziert, dass ein Ende des Fragmentes eine freie Nhel-Schnittstelle aufwies (sticky) und die andere Seite keine überhängende Enden zeigte (blunt). Die Ligation der so präparierten DNA-Sequenzen führten zum Reporter-Plasmid hPER1-luc.

### 2. Zellkultur

Humane Hepatomazellen (HUH-7) wurden in Dulbecco's minimum essential medium (DMEM) mit 10% fötalem Rinderserum in Gegenwart von Penicillin/Streptomycin (100 U/ml) und 2 mM L-Glutamin bei 37°C in einer 5%igen CO₂ Atmosphäre kultiviert. Die Transfektion erfolgte gemäß den Vorschriften des Herstellers Qiagen.

Die Testung erfolgt von jeweils 40.000 Zellen pro Well, worin die Zellen für 16 Stunden inkubiert und anschließend für 4 Stunden in einem Gesamtvolumen von 25 µl stimuliert wurden. Die zu untersuchenden Substanzen wurden für 20 min vorinkubiert und dann wurden 25 µl steady glo solution (Firma Promega) jedem Well zugesetzt und die Zellen lysiert. Die Lichtemission wurde mit einem Luminometer gemessen.

### 3. Induzierbarkeit des hPER1-Luc Konstruktes

Die Induzierbarkeit des Konstruktes wurde mit Hilfe von Forskolin getestet. Forskolin ist ein bekannter Induktor der hPER1 Expression. Forskolin führte zu einer konzentrationsabhängigen Induktion der Expression der Luziferase in HUH-7 Zellen.

### 4. Induzierbarkeit durch cAMP-Analoga

Es ist bekannt, dass Forskolin die cAMP-Spiegel in Zellen erhöht. Daher wurde das Zellpermeable und Proteinkinase A spezifische cAMP-Analog Sp-5,6-DCIcBiMPS, (5,6-dichlorobenzimidazole-1-β-D-ribofuranosyl-3',5'-monophosphorothioate, Sp-isomer) untersucht. Der Einfluss verschiedener Konzentrationen des cAMP-Analogs in Gegenwart von PMA (Phorbol 12-myristate 13-acetate) konnte nachgewiesen werden.

### 5. Screeningverfahren mit der erfindungsgemäßen Vorrichtung:

Zur Messung intrinsischer Luziferase-Aktitivität muß in einem ersten Schritt die zirkadiane "Slave" Kammer konstruiert und das System validiert werden. Hierzu muß im wesentlichen die Funktionalität der "Stempel" und die Varianzen des Messystems überprüft und gegebenenfalls optimiert werden.
I. HuH-7 und SH-SY5Y-Zellen (hPER1-Promoter-Luziferase transfiziert) werden angezogen und in eine erfindungsgemäße Vorrichtung mit 96 Vertiefungen (wells) plattiert. Die Zellen werden so ausplattiert, das zu Beginn der Messung 70% Konfluenz vorliegt, ca. 25000 bis 80000 Zellen pro well (abhängig vom Zelltyp) und pro 50-100 µl. Nach der Positionierung der Zellen in der zirkadianen "Slave" Kammer werden die Zellen unter konstantem Medium-Fluß (O₂ - gesättigtes, HEPES-gepuffertes Medium, 37°C, incl. 100 µM Na-Luziferin) gesetzt. Die von den Zellen emittierten Photonen werden von einer CCD-Kamera registriert und über 2-5 min Abstände integriert. Ohne weitere Beeinflussung des System werden in diesem Stadium die verwendeten Parameter optimiert:
   - verwendetes Medium (Puffersystem)
   - Flußrate des Mediums
   - minimale Konzentration von Luziferin
   - initiale Dichte der Zellen

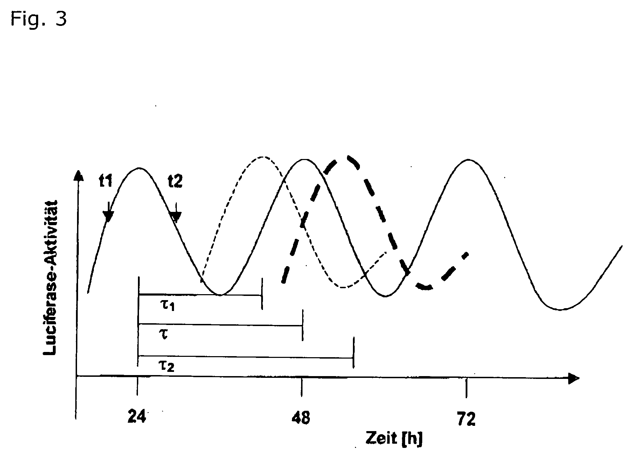

   Mit Hilfe dieser initialen Schritte wird ein zirkadianes Profil der bereits etablierten Zelllinien erstellt. Die spezifische Rhythmik der Zellen kann durch die intrinsischen Faktoren zur Regulation des zirkadianen Rhythmus und die Synchronisation der Phase der Zellen durch Trypsinisierung bzw. das Ausplattieren auf den Well-Schalen gemessen werden. Die Amplitude und/oder die Phasenlänge tau (τ) weist dabei zelltypspezifische Variationen auf und wird durch Auftragen der Luziferase-Aktivität gegen die Zeit ermitteln (Figur 3).
II. In einem weiteren Schritt werden über Schleifen bekannte Stimulatoren des zirkadianen Rhythmus eingelassen (Forskolin, sp-5,6-DCI-cBiMPS, PMA) und die Veränderung der Phasenlänge tau (τ) gemessen (Motzkus et al., 2000). Hierbei soll auch der ideale Zeitpunkt zur Beeinflussung des Systems bestimmt werden (τ = Max.), der für das anschließende "Screening" verwendet werden soll.
III. Eine weitere Validierung des Systems besteht im parallelen Betrieb der einzelnen Wells der Luminszenz-Messeinheit. Während dieser experimentellen Phase wird die "Betreibbarkeit" des Systems getestet, d.h. eine Gewährleistung findet statt, dass während eines Zeitraums von mindestens 4 Tagen alle gesetzten Parameter (Fluß, Medium, Temperatur, Lichtmessung etc.) in allen parallelen Kammern konstant bleiben.
IV. Nach der Einstellung aller essentiellen Parameter werden nach einem Vorlauf von ca. 24 h parallel bis zu 96 Fraktionen (incl. Kontrollen) Hämofiltrat-Äquivalentmengen über Weichen in die einzelnen Messkammern gegeben (Zeitpunkt der Stimulation gemäß II.). Die für das Screening verwendeten Fraktionen aus humanem Hämofiltrat und ihre Konzentrationen werden dabei gemäß DE3633707 (Forssmann, 1988) ausgewählt.

Das Ergebnis stellt sich dar wie in Figur 3: Diese zeigt das zirkadiane Profil einer bestimmten Zelllinie und die Veränderung der Phasenlänge tau (τ) nach der Applikation Rhythmus-modulierender Substanzen. Durch die intrinsische Rhythmizität der Zellen werden Reportergen-Aktivitäts-Maxima bestimmt, die circa alle 24 Stunden auftreten. Dabei haben verschiedene Zelllinien verschieden hohe Aktivitätsmaxima und auch Phasenlängen (τ). Durch die Applikation einer Hämofiltratfraktion, die eine Rhythmus-modulierende Substanz enthält, kommt es zur Verschiebung von τ: Bei Applikation zum Zeitpunkt t₁ erfolgt diese in Richtung zu früher (gestrichelte Kurve) oder zum Zeitpunkt t₂ in Richtung später (fett gedruckte gestrichelte Kurve) relativ zur intrinsischen Phasenlänge. Bei Applikation bei t₁ verkürzt sich die Phasenlänge τ zu τ₁ und bei Applikation zum Zeitpunkt t₂ verlängert sie sich zu τ₂.

Pro 96-Well-Platte werden ungefähr zwei Tage benötigt, um eine Phasenveränderung festzustellen. Aus diesem Grund ist es notwendig, dass das System möglichst unabhängig messen kann, so dass maximal vier Tage für einen kompletten Versuchsablauf benötigt werden. Ein initiales Screening einer Hämofiltratbank mit 400 Fraktionen mit n=3 dauert somit ungefähr vier Wochen. Natürlich kann das Screening erfindungsgemäß auch deutlich schneller und in größerem Maßstab durchgeführt werden, wenn eine größere Anzahl von Messungen parallel durchgeführt wird.

### Literaturverzeichnis:

AKIYAMA, M., KOUZU, Y., TAKAHASHI, S., WAKAMATSU, H., MORIYA, T., MAETANI, M., WATANABE, S., TEI, H., SAKAKI, Y. und SHIBATA S. (1999). Inhibition of light- or glutamate-induced mPer1 expression represses the phase shifts into the mouse circadian locomotor and suprachiasmatic firing rhythms. J Neurosci 19: 1115-21.
ALBRECHT, U., ZHENG, B., LARKIN, D., SUN, Z.S. und LEE, C.C. (2001). MPer1 and mper2 are essential for normal resetting of the circadian clock. J Biol Rhythms 16: 100-4.
ALBRECHT, U,., SUN, Z. S., EICHELE, G. und LEE, C.C. (1997). A differential response of two putative mammalian circadian regulators, mper1 and mper2, to light. Cell 91: 1055-64
ASCHOFF, J. (1981) A survey on biological rhythms. In: Biological Rhythms, Vol. 4, Handbook of Behavioral Neurobiology, Aschoff J. (ed. ), Plenum Press, New York.
BALSALOBRE, A., DAMIOLA, F. und SCHIBLER, U. (1998). A serum shock induces circadian gene expression in mammalian tissue culture cells. Cell 93: 929-937.
MOTZKUS, D., MARONDE, E., GRUNENBERG, U., LEE, C.C., FORSSMANN, W.G. und ALBRECHT, U. The human PER1 gene is transcriptionally regulated by multiple signaling pathways (2000). FEBS Lett 486: 315-9.
OISHI, K., SAKAMOTO, K., OKADA, T., NAGASE, T. und ISHIDA, N. (1998). Humoral signals mediate the circadian expression of rat period homologue (rPer2) mRNA in peripheral tissues. Neurosci Lett 256: 117-119.
SAKAMOTO, K., NAGASE, T., FUKUI, H., HORIKAWA, K., OKADA, T., TANAKA, H., SATO, K., MIYAKE, Y., OHARA, O., KAKO, K. und ISHIDA N. (1998). Multitissue circadian expression of rat period homolog (rPer2) mRNA is governed by the mammalian circadian clock, the suprachiasmatic nucleus in the brain. J Biol Chem 273: 27039-42.
SEILER, P., STÄNDKER, L., MARK, S., HAHN, W., FORSSMANN, W.G. und MEYER, M. (1999). Application of a peptide bank from porcine brain in isolation of regulatory peptides. J Chromatogr A 852: 273-83.
SILVER, R., LESAUTER, J., TRESCO, P.A. und LEHMAN, M.N. (1996). A diffusible coupling signal from the transplanted suprachiasmatic nucleus controlling circadian locomotor rhythms. Nature 382: 810-813.
SUN, Z.S., ALBRECHT, U., ZHUCHENKO, O., BAILEY, J., EICHELE, G. und LEE C.C. (1997). RIGUI, a putative mammalian ortholog of the Drosophila period gene. Cell 90: 1003-11.
VAN ESSEVELDT, K.E., LEHMAN, M.N. und BOER, G.J. (2000). The suprachiasmatic nucleus and the circadian time-keeping system revisited. Brain Res Brain Res Rev 33: 34-77.
YAMAZAKI, S., NUMANO, R., ABE, M., HIDA, A., TAKAHASHI, R., UEDA, M., BLOCK, G.D., SAKAKI, Y., MENAKER, M. und TEI, H. (2000). Resetting central and peripheral circadian oscillators in transgenic rats. Science 288: 682-685.

### Bezugszeichenliste:

- 10: Dunkelkammer
- 12: Kammer
- 14: Kammer
- 16: Haltevorrichtung, Halteeinrichtung
- 18: Probenträger
- 20: Vertiefung, Wells
- 22: Verschlußeinrichtung
- 24: Einlaßöffnung, Öffnungen
- 25: Einlaßöffnung
- 26: Auslaßöffnung, Öffnungen
- 28: Schlauch
- 30: Schlauch
- 32: Kanäle
- 34: Kanäle
- 36: Pumpe, Medium-Fördereinrichtung
- 40: Ventileinrichtung
- 42: Pumpe
- 44: Meßeinrichtung, CCD-Kamera
- 46: Leitung
- 48: Auswerteeinrichtung
- 50: Zellen
- 52: Heizeinrichtung, Temperiervorrichtung
- 54: Probenspritze

## Patentansprüche

1. Vorrichtung zur zeitaufgelösten Untersuchung von Prozessen mit einem in einer Dunkelkammer (10) angeordneten Probenträger (18) mit einer Vielzahl von Vertiefungen (20), Verschlußeinrichtungen (22) zum Verschließen der Vertiefungen (20), wobei jede Verschlußeinrichtung (22) eine mit der entsprechenden Vertiefung (20) verbundene Einlaßöffnung (24) und eine Auslaßöffnung (26) zum Zu- und Abführen von Medium in bzw. aus der Vertiefung (20) aufweist, und einer in der Dunkelkammer (10) angeordneten Meßeinrichtung (44) zum Messen der von den Zellen (50) abgegebenen Photonen.

2. Vorrichtung nach Anspruch 1, **dadurch gekennzeichnet, dass** für jede Vertiefung (20) eine einzelne Verschlußeinrichtung (22) vorgesehen ist.

3. Vorrichtung nach einem der Ansprüche1 oder 2, **dadurch gekennzeichnet, dass** die Verschlußeinrichtungen (22) in die Vertiefungen (20) hineinragen.

4. Vorrichtung nach einem der Ansprüche 1 bis 3, **dadurch gekennzeichnet, dass** in der Dunkelkammer (10) eine Halteeinrichtung (16) zum lagegenauen Aufnehmen des Probenträgers (18) bezüglich der Meßeinrichtung (44) vorgesehen ist.

5. Vorrichtung nach einem der Ansprüche 1 bis 4, **dadurch gekennzeichnet, dass** die Meßeinrichtung (44) auf der den Verschlußeinrichtungen (22) gegenüberliegenden Seite des Probenträgers (18) angeordnet ist.

6. Vorrichtung nach einem der Ansprüche 1 bis 5, **dadurch gekennzeichnet, dass** als Meßeinrichtung (44) eine CCD-Kamera vorgesehen ist, wobei die CCD-Kamera bezüglich der Vertiefungen (20) derart angeordnet ist, dass eine definierte Anzahl von Pixeln jeweils einer Vertiefung (20) zugeordnet ist.

7. Vorrichtung nach einem der Ansprüche 1 bis 6, **dadurch gekennzeichnet, dass** jede Einlaßöffnung (25) und jede Auslaßöffnung (26) über ein gesondertes Fluidelement (28) mit einer Medium-Fördereinrichtung (36) verbunden ist.

8. Vorrichtung nach einem der Ansprüche 1 bis 7, **dadurch gekennzeichnet, dass** in der Dunkelkammer (10) eine Temperiervorrichtung (52) vorgesehen ist.

9. Verwendung der Vorrichtung nach einem der Ansprüche 1 bis 8 zur zeitaufgelösten Analyse von Prozessen.

10. Verwendung nach Anspruch 9 zur zeitaufgelösten Untersuchung von zellulären und/oder biochemischen Prozessen, insbesondere unter physiologischen Bedingungen.

11. Verwendung nach Anspruch 9 oder 10, **dadurch gekennzeichnet, dass** die zellulären und/oder biochemischen Prozesse die zelluläre Antwort auf äußere Stimuli, die zelluläre Antwort auf äußere Bedingungen, der zirkadiane Rhythmus von Zellen, Apoptose, die Expression von Genen, die Funktion von Promotern, Änderungen von Protein/Proteln-Wechselwirkungen, Phosphorylierung und Dephopsphorylierung von Signaltransduktionsmediatoren, Differenzierungs- oder Wachstumsprozesse, die Veränderungen von speziellen Markergenen für Differenzierungs- und Wachstumsprozesse, Proteindislokation, Änderungen von Proteinkonzentrationen in bestimmten Zellkompartimenten und/oder Proteintranslokation sind.

12. Verfahren zur zeitaufgelösten Untersuchung von zellulären und/oder biochemischen Prozessen unter Verwendung einer Vorrichtung nach einem der Ansprüche 1 bis 8, **dadurch gekennzeichnet, dass** in den Vertiefungen (20) Zellen positioniert werden, flüssige Medien durch die Einlaßöffnungen (24) den Vertiefungen (20) zugeführt werden und durch die Auslaßöffnungen (26) aus den Vertiefungen abgeführt werden, und dass die Abgabe von Strahlung, insbesondere von Photonen, aus den Vertiefungen (20) mit Hilfe der Messeinrichtung (44) verfolgt wird.

13. Verfahren nach Anspruch 12, **dadurch gekennzeichnet, dass** die Medien unter konstantem Fluß zugeführt werden und/oder dass das Verfahren automatisiert ist.

14. Verfahren nach einem der Ansprüche 12 oder 13, **dadurch gekennzeichnet, dass** die zellulären oder biochemischen Prozesse solche gemäß Anspruch 11 sind.

15. Verfahren nach einem der Ansprüche 12 bis 14, **dadurch gekennzeichnet, dass** zeitaufgelöst untersucht wird, ob und wie in den Medien enthaltene Substanzen oder Substanzgemische zelluläre Prozesse auslösen oder beeinflussen.

16. Verfahren nach Anspruch 15, **dadurch gekennzeichnet**, das die Substanzen oder Substanzgemische Peptide oder Peptidgemische sind oder enthalten.

17. Verfahren nach Anspruch 16, **dadurch gekennzeichnet, dass** die Peptide oder Peptidgemische solche aus Hemofiltratbanken, Plazentabanken oder anderen Peptidbanken menschlichen Ursprungs sind.

18. Verfahren nach Anspruch 16, **dadurch gekennzeichnet, dass** die Peptide oder Peptidgemische aus tierischen, insbesondere bovinen oder porcinen Geweben oder aus humanen oder tierischen Zelllinien oder deren Zellkulturüberständen isoliert worden sind.

19. Verfahren nach einem der Ansprüche 12 bis 17, **dadurch gekennzeichnet, dass** die Zellen mit einem Konstrukt aus dem Promoter des humanen Period1-Gens und einem Reportergen transfiziert sind, mit Substanzen oder Substanzgemischen inkubiert werden und jeweils die Expression des Reportergens bestimmt wird.

20. Verfahren nach Anspruch 18, **dadurch gekennzeichnet, dass** die Zellen mit einem Konstrukt (hPER1-luc) aus dem Promoter des humanen Period1-Gens und dem Reportergen Luziferase transfiziert sind und dass die Medien Luziferin enthalten.

21. Verwendung des Verfahrens nach einem der Ansprüche 12 bis 19 zur Identifizierung von schlafmodulierenden und neuroaktiven Substanzen in menschlichen Zelllinien, zum Screening von Substanzbibliotheken, insbesondere Hemofiltratbanken oder Plazentabanken, zum Screening von potentiellen Wirkstoffen gegen schlafassozierten Erkrankungen, ausgewählt aus der Gruppe umfassend "Jet-Lag", Folgen von anhaltender Schichtarbeit und 'seasonal affective disorders' (SAD), Tag-Müdigkeit mit Schlafanfällen (hypersomnia), Schlafstörungen, Fettsucht, Kohlenhydrat-Begierde, Symptomen die der Überwinterung ähneln, Depressionen, Psychosen, Veränderungen der Melatonin- und Cortison-Sekretion, Verkürzung der REM-Schlaf-Phase und Erkrankungen der Physis und des Gemüts, die im Zusammenhang mit zirkadianen Störungen beim Menschen stehen.
